# FASCICULE DE BREVET EUROPEEN

(11) **EP 0 303 538 B1**
(45) Date de publication et mention de la délivrance du brevet: **05.05.1993**
(21) Numéro de dépôt: 88402068.6
(22) Date de dépôt: 09.08.1988
(51) Int. Cl.: C07K 15/14, C07K 3/28, C12P 21/02, A61K 37/02

(54) **Protéine à activité vasculaire, dérivée des monocytes et ses analogues, procédé pour son extraction et leurs utilisations en thérapeutique et pour la préparation d'anticorps**
Von Monozyten abstammendes gefässwirksames Protein und dessen Analogen, Verfahren zu seiner Gewinnung und ihre Verwendungen für therapeutische Zwecke und zur Antikörperherstellung
Protein with a vascular activity, derived from monocytes and its analogues, process for its extraction and their uses as therapeutic agents and for the preparation of antibodies

(30) Priorité: 12.08.1987 FR 8711490
(43) Date de publication de la demande: 15.02.1989
(73) Titulaire: INSTITUT DES VAISSEAUX ET DU SANG, F-75010 Paris (FR)
(72) Inventeur: Wautier, Jean-Luc, F-75019 Paris (FR)
(74) Mandataire: Ayache, Monique

(56) Documents cités:
- PROCEEDINGS OF THE NATIONAL ACADEMY SCIENCES USA, vol. 83, mai 1986, pages 3321-3325; M. TODD BROWN et al.: "Platelets contain a peptide inhibitor of endothelial cell replication and growth"
- ARTERIOSCLEROSIS - A JOURNAL OF VASCULAR BIOLOGY AND DISEASE, vol. 5, janvier 1985, pages 110-115, Dallas, Texas, US; B.L. SCHUMACHER et al.: "Smooth muscle cells produce an inhibitor of endothelial cell growth"
- CLINICAL IMMUNOLOGY AND IMMUNOPATHOLOGY, vol. 39, 1986, pages 242-255, Academic Press, Inc.; M.B. KAHALEH et al.: "Human monocyte modulation of endothelial cells and fibroblast growth: Possible mechanism for fibrosis"
- THROMBOSIS AND HAEMOSTASIS, vol. 58, no. 1, 1987, page 106; F. LIOTE et al.: "Inhibition of endothelial cell proliferation by normal human monocytes"
- SIXTH INTER-WASHINGTON SPRING SYMPOSIUM, résumé 160a, 1986, editor L.L. Gallo; A. COURILLON MALLET: "Monocyte-derived endothelial cell inhibitory factor (MECIF)"

## Description

L'invention a pour objet une famille de protéines, notamment de glycoprotéines, à activité vasculaire, inhibitrices de la prolifération des cellules endothéliales, en particulier un facteur dérivé des monocytes, un procédé pour l'extraction de ce facteur, et les applications de ces protéines.

La régulation de la croissance des cellules vasculaires est un facteur important dans la genèse et le développement de l'athérosclérose et de la néoangiogenèse. Récemment, on a porté une attention toute particulière aux facteurs de croissance qui stimulent la prolifération des cellules endothéliales, des cellules des muscles lisses et des fibroblastes. On n'a caractérisé qu'un petit nombre de facteurs qui sont capables d'inhiber la prolifération des cellules vasculaires.

De nombreux chercheurs ont récemment concentré leur intérêt sur le rôle des monocytes circulants dans la régulation de la croissance des cellules des parois vasculaires. La participation des monocytes à de nombreux processus pathologiques impliquant la paroi des vaisseaux sanguins tels que la vascularite, l'athérosclérose, l'inflammation et les métastases cancéreuses a été récemment signalée (références 1 et 2). Les facteurs de régulation de la croissance tiennent des rôles importants dans de tels cas. Des facteurs favorisant la croissance, dérivés des monocytes MDGF (Monocyte-derived Growth Promoting Factors) ont été isolés et caractérisés (références 3 à 11).

Les résultats des travaux préliminaires du demandeur (référence 12) qui sont en accord avec les résultats obtenus par Kahaleh et coll. (référence 13), montrent qu'outre les facteurs favorisant la croissance, les monocytes peuvent également produire des facteurs inhibiteurs.

Le demandeur a en particulier établi que les monocytes humains normaux produisent un facteur inhibiteur de la prolifération des cellules endothéliales humaines, auquel il a donné le nom de MECIF (Monocyte-derived Endothelial Cell Inhibitory Factor : facteur inhibiteur des cellules endothéliales, dérivé des monocytes).

Le demandeur a maintenant mis au point un procédé d'extraction permettant d'obtenir le MECIF a un degré de pureté tel qu'il a été possible de l'analyser.

Selon l'un de ses aspects, l'invention a pour objet un procédé pour l'extraction d'un facteur inhibiteur des cellules endothéliales, dérivé des monocytes ou MECIF, caractérisé en ce qu'il comprend essentiellement les étapes consistant à :
1°) cultiver des monocytes humains normaux,
2°) faire passer le surnageant de culture filtré à travers une colonne en polysaccharide réticulé, équilibrée à pH 8 environ,
3°) faire passer ensuite ce surnageant à travers une colonne de gel d'agarose phénylé et réticulé, équilibrée à pH 8 environ, en développant avec un gradient décroissant de sulfate d'ammonium,
4°) faire passer les fractions actives obtenues sous 3°) à travers une colonne du type de celle utilisée sous 2°), équilibrée à pH 8 environ,
5°) faire passer l'éluat obtenu sous 4°) à travers une colonne échangeuse d'ions de type diéthylaminoéthylée (DEAE), en développant avec un gradient croissant de chlorure de sodium,
6°) faire passer les fractions obtenues sous 5°) à travers une colonne de chromatographie d'exclusion basse pression, équilibrée au moyen d'un milieu de culture de cellules, et
7°) recueillir le produit purifié, éventuellement après avoir soumis les fractions actives obtenues sous 6°) à une chromatographie liquide haute pression.

Les monocytes humains normaux sont isolés du sang, soit par centrifugation différentielle, soit par cytaphérèse puis par adhésion sélective dans des boîtes de plastique recouvertes ou non de fibronectine. Le détail de cette technique est décrit dans la partie expérimentale qui suit.

Les monocytes sont avantageusement cultivés pendant environ 24 heures.

La colonne en polysaccharide réticulé peut être par exemple du type de celle commercialisée sous la dénomination Sephadex® G-25 par la Société Pharmacia Fine Chemicals (Uppsala, Suède).

La colonne de gel d'agarose phénylé et réticulé peut être par exemple du type de celle commercialisée sous la dénomination Phenyl Sepharose® CL 4B par la même société Pharmacia.

La colonne échangeuse d'ions de type DEAE peut être par exemple celle commercialisée sous la dénomination DEAE-Trisacryl® LS par la Société IBF (Gennevilliers, France).

La colonne de chromatographie d'exclusion basse pression peut être par exemple celle commercialisée sous la dénomination Trisacryl® GF-05, par la même société IBF.

La chromatographie liquide haute pression (HPLC) peut être effectuée sur une colonne commercialisée sous la dénomination TSK-250 par la Société Bio-Rad (Munich, RFA).

Un exemple détaillé, non limitatif, de mise en oeuvre de ce procédé est donné dans la partie expérimentale du présent mémoire, pour mieux l'expliquer encore.

Selon une variante, le MECIF peut être isolé à partir de surnageants de lignées cellulaires leucémiques monocytoïdes comme la lignée HL 60 (référence 14), après stimulation par l'acide rétinoïque ou mieux encore par le DMSO.

Le MECIF et ses dérivés dont il sera question plus loin peuvent également être obtenus par les techniques du génie génétique, à partir de la séquence peptidique connue, avec isolement de l'ARNₘ correspondant.

L'analyse du MECIF purifié obtenu a permis d'établir qu'il s'agit d'une glycoprotéine de masse moléculaire apparente de 64-70 kd.

Cette glycoprotéine comporte 568 résidus d'acides aminés qui se répartissent selon les proportions approximatives suivantes :
Asp : 80 ; Thr : 23,06 ; Ser : 29,40 ; Glu : 56,5 ; Pro : 27,70 : Gly : 38,98 ; Ala : 42,88 ; Val : 36,16 ; Met : 4,83 ; Ile : 18,79 ; Leu : 50,92 ; Tyr : 20,15 ; Phe : 27,16 ; GlcNH₂ : 3,10 ; Lys : 60,87 ; His : 13,81; Arg : 27,60.

Sa séquence peptidique N-terminale est :
Ser-Pro-Glu-Leu-Thr-Phe-Arg-X-Y-Thr-Ile-Ser-
où X et Y, qui peuvent être identiques ou différents, représentent chacun Trp ou Asp.

Cette glycoprotéine comporte des sucres aminés et des sucres non aminés.

Elle peut être conservée à 4°C pendant 2 mois ou, pour des périodes plus longues, à -80°C ou sous forme lyophilisée.

La glycoprotéine ainsi définie constitue un autre objet de l'invention.

L'invention a également pour objet une protéine, de préférence glycosylée, de poids moléculaire compris entre environ 0,6 et 200 kd, comportant la séquence :
-Ser-Pro-Glu-Leu-Thr-Phe-

De préférence, cette séquence est N-terminale.

De préférence encore, la séquence en question est :
-Ser-Pro-Glu-Leu-Thr-Phe-Arg-X-Y-Thr-Ile-Ser-
où X et Y, qui peuvent être identiques ou différents, représentent chacun Trp ou Asp.

X et Y sont de préférence différents et, de préférence encore, X représente Trp et Y représente Asp.

Une telle protéine est avantageusement préparée par mise en oeuvre des techniques classiques du génie génétique.

Le MECIF est actif sur les cellules endothéliales humaines à des concentrations de l'ordre du picogramme par millilitre et est peu actif sur les cellules endothéliales de porc. Il ne modifie pas de manière sensible la croissance des cellules des muscles lisses, des artères humaines, ni des fibroblastes de peau adulte ou des fibroblastes de poumon embryonnaire.

Sur les cellules endothéliales humaines son action est détectable après 6 heures de contact. Elle atteint un plateau au bout de 12 heures et est stable 48 heures.

Son action est partiellement réversible (50 %) par l'addition d'un facteur de croissance (ECGF : Edothelial Cell Growth Factor : facteur de croissance des cellules endothéliales).

Le MECIF n'est pas cytotoxique à la concentration de 5 mg/ml.

Le MECIF et les (glyco)protéines en dérivant, définies ci-dessus, possédant les mêmes propriétés, peuvent être transformés, en présence d'adjuvants et/ou excipients usuels, en formes galéniques destinées à l'application locale cutanée, à l'injection intra-articulaire, à l'injection sous-cutanée ou à la perfusion intraveineuse, ou en collyre, notamment.

Le MECIF et ses dérivés possédant les mêmes propriétés peuvent être utilisés en thérapeutique, notamment :
- en ophtalmologie dans le traitement des rétinopathies diabétiques, de la néovascularisation, de la dégénérescence maculaire sénile ;
- dans le traitement de la maladie vasculaire périphérique ou artérite, dans la prévention de l'obstruction des prothèses par prolifération endothéliale ;
- dans le traitement de la maladie veineuse pour empêcher la prolifération des néocapillaires, dans le traitement de la maladie veineuse des membres inférieurs et de la couperose ;
- dans le traitement de la maladie vasculaire tumorale: angiomes avec prolifération vasculaire, syndrome de Kaposi, néoangiogenèse tumorale (tumeurs solides, reins, foie ...) ;
- dans le traitement de la maladie inflammatoire : arthrite rhumatoïde.

La forme d'administration et la dose dépendent essentiellement de la maladie à traiter, d'une part et des caractéristiques particulières du patient, d'autre part. Elles peuvent être déterminées par l'Homme du métier sur la base de ses connaissances usuelles.

En outre, le MECIF peut être utilisé pour produire, selon des techniques usuelles, des anticorps polyclonaux ou monoclonaux spécifiques qui peuvent être utilisés pour déterminer les taux de MECIF dans la pathologie vasculaire et tumorale, rhumatismale et inflammatoire.

Pour ce faire, on peut avantageusement avoir recours à la techniques ELISA, notamment telle que décrite à la référence 15.

Les dérivés du MECIF décrits plus haut peuvent également être utilisés pour produire des anticorps.

La description détaillée qui suit d'un exemple d'extraction du MECIF et de son étude physico-chimique et pharmacologique est destinée à mieux expliquer l'invention sans en limiter la portée.

### PARTIE EXPERIMENTALE

### I. Préparation d'un surnageant de culture de monocytes :

On utilise un échantillon de sang humain complet citraté provenant de donneurs normaux en bonne santé. Les cellules mononucléées sont d'abord séparées par centrifugation à gradient de densité puis les monocytes sont isolés par une méthode d'adhésion sélective, c'est-à-dire en ensemençant les cellules mononucléées sur des boîtes de culture en matière plastique (référence 16). Les cellules mononucléées ayant adhéré récupérées après cette mise en oeuvre sont constituées par 95 ± 3 % de monocytes, selon la détermination effectuée par coloration spécifique à l'estérase et marquage des cellules par des anticorps monoclonaux spécifiques pour les monocytes et les lymphocytes (référence 16). La contamination par des plaquettes est comprise entre 5 et 10 plaquettes par monocyte.

Les monocytes sont ensuite incubés dans du milieu de culture M-199 provenant de la société GIBCO, additionné de 2,5 % de sérum de veau foetal (FCS) pendant 24 heures à 37°C dans une atmosphère à 5 % de CO₂. A la fin de cette période, le surnageant de culture est recueilli et filtré au moyen d'un filtre de 0,2 µ de porosité. Toutes les étapes qui précèdent sont mises en oeuvre dans des conditions stériles.
**Note :** Les effets de différents produits chimiques sur la production du MECIF ont été étudiés en ajoutant ces produits aux monocytes au début de l'incubation.

### II. Purification de la protéine :

### Matériels

Les supports de chromatographie, à savoir le Sephadex® G-25, la Phényl Sépharose® CL4B proviennent de Pharmacia Fine Chemicals (Uppsala, Suède), le DEAE-Trisacryl® LS et le Trisacryl® GF-05, proviennent d'IBF (Gennevilliers, France) et la colonne HPLC TSK-250 provient de Bio-Rad (Munich, RFA). Tous les produits chimiques utilisés sont de qualité "réactif".

A chaque étape du processus de purification, chaque fraction est testée en triple exemplaire dans l'essai de prolifération des cellules endothéliales pour détecter la fraction contenant l'activité inhibitrice.

Dans des essais séparés, on fait passer plusieurs portions d'un litre de surnageant de culture de monocytes à travers une colonne de Sephadex G-25 (4,4 X 70 cm) qui est équilibrée avec du tampon Tris 10 mM, pH 8,0. Les fractions actives rassemblées sont ensuite traitées par du sulfate d'ammonium solide à 30 % de saturation et, après avoir été centrifugé, on fait passer ensuite le surnageant à travers une colonne de Phényl Sépharose (3,2 X 33 cm) équilibrée avec du tampon Tris 10 mM, pH 8,0 contenant du sulfate d'ammonium à 30 % de saturation. Après mise en place de l'échantillon et rinçage adéquat avec du tampon d'équilibrage, la colonne est développée au moyen d'un gradient linéaire de sulfate d'ammonium allant de 30 jusqu'à 0 %, dans du tampon Tris 10 mM, pH 8,0. Les fractions actives sont rassemblées et on les fait passer à travers une colonne de Sephadex G-25 comme décrit plus haut, avant de faire une séparation supplémentaire au moyen d'une colonne de DEAE Trisacryl-LS (1,5 X 8 cm) avec un gradient linéaire de NaCl allant de 0 jusqu'à 1 M dans du tampon Tris 10 mM, pH 8,0. Avant d'être examinées en ce qui concerne leur activité biologique sur les cellules vasculaires en culture, toutes les fractions de chromatographie doivent être passées à travers une colonne de Trisacryl GF-05, (2,4 X 10 cm), équilibrée avec un milieu de culture, à savoir du tampon de Hanks (provenant de l'Institut Pasteur, Paris, France). Le facteur de purification final jusqu'à la chromatographie sur la colonne de GF-05 est de 570 fois. Pour vérifier que la protéine isolée après les chromatographies sur colonnes DEAE et GF-05 représente la protéine MECIF et pour déterminer le poids moléculaire de cette protéine, la fraction active est ensuite soumise à un autre processus de chromatographie sur une colonne HPLC TSK-250 et analysée par électrophorèse SDS PAGE (Sodium Dodecyl Sulfate - Poly Acrylamide Gel Electrophoresis : électrophoresis dodécylsulfate de sodium - gel de polyacrylamide).

La concentration en protéine est déterminée sur la fraction pure au moyen de la composition en amino-acides et par mesure à l'auto-analyseur.

La composition en amino-acides donnée plus haut est déterminée à l'aide d'un séquenceur.

L'analyse à l'autoanalyseur d'un échantillon du produit a permis de déterminer les concentrations suivantes en amino-acides (valeurs en picomoles) :
Asp : 20,079 ; Thr : 5,79 ; Ser : 7,38 ; Glu : 14,18 ; Pro : 6,95 ; Gly : 9,78 ; Ala : 10,764 ; Val : 9,078 ; Met : 1,212 ; Ile : 4,716 ; Leu : 12,781 ; Tyr : 5,058 ; Phe : 6,819 ; GlcNH₂ : 0,778 ; Lys : 15,279 ; His : 3,48 ; Arg : 6,92.

On en a déduit les nombres approximatifs de résidus de ces différents amino-acides donnés plus haut.

### III. Essai de culture cellulaire et de prolifération :

Des cellules endothéliales vasculaires humaines sont recueillies à partir du cordon ombilical et mises en culture selon une technique décrite précédemment (référence 17). Les fibroblastes de peau humaine et de poumon embryonnaire, sont un cadeau de A. Macieira Coehlo (INSERM U-50, Villejuif, France) et les cellules de muscles lisses fémoraux humains sont un cadeau de J. Larrue (INSERM U-8, Pessac, France). Les cellules endothéliales aortiques porcines ont été fournies par L. Drouet (INSERM U-150, Paris, France). La (méthyl-³H)-thymidine (5 Ci/mmole) provenait du Commissariat à l'Energie Atomique, France. Les cellules vasculaires en culture, marquées à la thymidine, ont été recueillies en utilisant un "collecteur" (harvester) de cellules (Skatron, Lier, Norvège).

L'activité biologique du MECIF a été testée en triple exemplaire par incorporation de la (méthyl-³H)-thymidine dans l'ADN de cellules vasculaires en culture. Pour ce faire, 12 à 15000 cellules dans du milieu de culture M-199 additionné de 10 % de sérum de veau foetal sont ensemencées dans chaque puits de culture d'une boîte multipuits et incubées pendant 24 heures à 37°C en présence de MECIF et sous une atmosphère à 5 % de CO₂. Les cellules cultivées sont ensuite incubées pendant 16 heures avec de la (méthyl-³H)thymidine (1 µCi/puits). A la fin de cette période, le milieu est éliminé et, après exposition à de la collagénase, les cellules sont recueillies sur des filtres en fibres de verre en utilisant un "collecteur" de cellules. Les cellules endothéliales humaines et porcines employées pour l'étude de prolifération provenaient de cultures primaires, alors que les cellules de muscles lisses et les fibroblastes provenaient des huitième et treizième passages, respectivement.

### Résultats :

### Production du MECIF par des monocytes humains normaux en culture :

On a trouvé que les monocytes de sang humain isolés libèrent un facteur appelé MECIF qui peut inhiber la prolifération des cellules endothéliales de la veine ombilicale humaine, comme le montre l'incorporation de la (méthyl-³H)thymidine dans ces cellules. La production du MECIF par les monocytes en culture atteint son maximum après 24 heures d'incubation puis décline après cette période. Toutefois, le MECIF présent dans le milieu après les 24 premières heures pourrait rester stable pendant au moins 72 heures. La production du MECIF est dépendante du nombre de monocytes et des concentrations en protéines du sérum dans le milieu de culture. L'expression maximale du MECIF a été observée lorsque la concentration en sérum de veau foetal dans le milieu de culture des monocytes était de 2,5 %.

Pour exclure la possibilité que les cellules contaminantes soient à l'origine de l'expression du MECIF, des milieux de culture de plaquettes ou de lymphocytes aux concentrations normalement trouvées dans la préparation de monocytes ont été testés sur les cellules endothéliales en culture en employant un processus similaire à celui décrit pour les monocytes. Les résultats ont montré au contraire un accroissement de la synthèse d'ADN dans les cellules endothéliales, suggérant ainsi que la production du MECIF est directement attribuable aux monocytes.

Le traitement des monocytes en culture par le cycloheximide élimine complètement la libération du MECIF dans le milieu de culture. Par ailleurs, le traitement par l'indométhacine, un inhibiteur de la cyclo-oxygénase, ne conduit pas à une altération de l'expression du MECIF. Cela suggère la nécessité de la synthèse des protéines, mais non du métabolisme de l'acide arachidonique, pour l'expression du MECIF.

L'activation des monocytes par l'endotoxine [LPS (lipopolysaccharide), Escherichia coli, 10 µg/ml] ou par la fMLP (formyl-Méthionyl Leucine Phénylalanine, 0,1 µM) ne favorise pas la production du MECIF.

Le stockage d'un milieu de culture contenant du MECIF à 4°C n'a pas d'effet sur la bioactivité et le milieu est stable pendant 5 minutes à 56°C.

La bioactivité du MECIF n'est pas neutralisée par l'aprotinine, un inhibiteur de protéase.

D'autres observations ont montré que le MECIF n'exerce pas sa bioactivité sur les cellules endothéliales au moyen d'une cytotoxicité directe, étant donné qu'il ne provoque pas la libération de ⁵¹Cr à partir des cellules endothéliales préalablement marquées.

### Purification de la protéine :

Après l'étape de purification sur Phényl Sépharose, l'activité du MECIF sur les cellules endothéliales a été observée dans le pic élué à environ 14 % de sulfate d'ammonium. Les autres fractions présentaient une activité négligeable. L'étape de purification supplémentaire avec le DEAE Trisacryl-LS a séparé le MECIF des autres protéines. La fraction contenant le MECIF a été éluée dans le premier pic et l'analyse par électrophorèse sur gel a montré qu'il s'agissait d'une bande unique.

L'activité de cette protéine a été encore confirmée telle que présente dans les fractions du pic unique, après avoir subi la chromatographie HPLC TSK-250. En outre, en faisant passer le milieu de culture de monocytes contenant le MECIF brut à travers la colonne de TSK-250, les fractions actives ont été trouvées dans le même volume que les fractions actives provenant de la chromatographie sur DEAE.

Des essais pour introduire des chromatographies sur Héparine Sepharose® et sur Cibacron Blue F3GA Sepharose® dans le processus de purification ont été également effectués. Toutefois, on a trouvé que le MECIF ne pouvait pas être retenu par l'un ou l'autre de ces supports de chromatographie.

### Propriétés du MECIF purifié :

Comme déterminé par électrophorèse SDS/PAGE dans des conditions non réduites et avec coloration par du Bleu de Coumassie, le MECIF purifié après chromatographie sur DEAE-Trisacryl LS migre sous forme d'une bande unique large ayant un poids moléculaire apparent de 64 kd. Dans des conditions réduites, il apparaît sous forme de deux bandes avec une bande majeure d'environ 70 kd et une seconde d'environ 68 kd. La chromatographie liquide haute pression par TSK-250 (60 X 0,75 cm) révèle un pic unique correspondant à un poids moléculaire d'environ 66 kd.

La coloration du gel d'électrophorèse SDS/PAGE avec du nitrate d'argent ne révèle pas de bandes supplémentaires comme cela avait été observé avec la coloration par le Bleu de Coumassie. La réaction acide périodique-Schiff (PAS) est positive, démontrant que le MECIF est une glycoprotéine. Lorsque le surnageant des monocytes est soumis à une chromatographie liquide haute pression (sur TSK-250), l'activité inhibitrice se retrouve dans les fractions (poids moléculaire : 66 kd) éluées dans la même volume que l'était le MECIF pur, ce qui montre que le MECIF correspond à l'activité inhibitrice présente dans le surnageant de monocytes.

### Activité régulatrice de la croissance :

On a trouvé que l'inhibition de l'incorporation de la (méthyl-³H)thymidine est dépendante de la concentration du milieu de culture contenant le MECIF. La dilution de ce milieu à partir de sa concentration d'origine jusqu'à 64 fois provoque la diminution de l'effet inhibiteur de 80 à 30 % par rapport au témoin (n = 15). Une concentration de 14 pg de MECIF produit un effet inhibiteur de 81 ± 2,5 % (X ± SEM ; SEM = erreur standard de la moyenne) vis-à-vis de l'incorporation de la thymidine en présence de sérum de veau foetal (2,5 à 10 %) (n = 16). Les effets régulateurs de la croissance sur les cellules endothéliales vasculaires sont dépendants de la concentration en MECIF. Aux mêmes concentrations, le MECIF n'influence pas de façon marquante l'incorporation de thymidine dans les fibroblastes tant de peau humaine normale (113 ± 5 %, n = 6) que provenant de poumon embryonnaire (128 ± 6 %, n = 8). Dans les mêmes conditions, les cellules de muscles lisses et d'artères humaines ne sont pas affectées (108 ± 8 %, n = 12). Une inhibition de 28 ± 7 % (n = 12) est observée avec les cellules endothéliales porcines mais dans une mesure beaucoup moins importante.

Une étude au cours du temps a montré que la synthèse d'ADN est supprimée par le MECIF à partir de 6 heures d'incubation et atteint son plateau après 12 heures d'incubation. De plus, l'effet du MECIF dans l'inhibition de la synthèse d'ADN est parallèle à la réduction de la prolifération de cellules, ce qui a été déterminé par des comptages de cellules sous un microscope optique.

### Test de réversibilité :

Les cellules endothéliales ont d'abord été cultivées en présence de MECIF pendant 24 heures puis l'incorporation de la (méthyl-³H) thymidine à une partie de la culture cellulaire a été mesurée après 16, 72 et 120 heures. Tandis que dans deux autres cultures cellulaires, après cette incubation de 24 heures, on a poursuivi la culture des cellules en présence soit de 20 % de sérum de veau foetal, soit de 25 µg d'ECGF (facteur de croissance des cellules endothéliales) pendant d'autres périodes de 16, 72 et 120 heures. Les résultats ont été exprimés en pourcentage d'incorporation de la (méthyl-³H) thymidine dans ces cellules à la fin de chaque incubation, par comparaison avec les cellules cultivées en l'absence de MECIF.

Après le même test avec le TGFβ, on a trouvé que la réversibilité de la croissance des cellules endothéliales était plus facile à obtenir après exposition des cellules endothéliales au MECIF qu'après son exposition au TGFβ.

### Etude comparative entre l'activité du TGFβ et l'activité du MECIF.

Comme décrit par le fabricant (R & G, Minneapolis, Mn, E.U.A.), l'inventeur a trouvé que 40 µl (équivalant à 100 µg d'IgM) d'anticorps polyclonaux spécifiques dirigés contre le TGFβ humain (R & G) neutralisaient l'activité biologique de 2 ng/ml de TGFβ (Calbiochem, La Jolla, Ca). Différentes concentrations (2 à 12 µg/ml) de MECIF de pureté intermédiaire (après chromatographie sur DEAE) ont été utilisées et la quantité qui donnait 50 % d'inhibition de la prolifération des cellules endothéliales a été comparée à celle du TGFβ qui produisait aussi 50 % d'inhibition de la croissance (2 ng/ml). Le MECIF ou le TGFβ a été incubé avec les anti TGFβ pendant 1 heure à la température ambiante avant d'être testé quant à ses effets inhibiteurs. Ces anti TGFβ ne neutralisaient pas les effets inhibiteurs du MECIF sur la croissance des cellules endothéliales.

En observant les cellules endothéliales sous un microscope optique, la présence du MECIF dans leur milieu de culture provoque des changements de morphologie des cellules qui prennent une forme allongée. Toutefois, comme le démontre une étude en immunofluorescence et des observations en microscopie électronique à transmission, les cellules conservent encore leurs marqueurs spécifiques, c'est-à-dire le facteur de Willebrand et les corps de Weibel Palade.

Par ailleurs, lorsque le TGFβ a inhibé la prolifération des cellules endothéliales, il n'a pas modifié la morphologie des cellules.

### DISCUSSION

Les résultats présentés dans cette partie expérimentale montrent l'aptitude des monocytes humains normaux en culture à libérer un facteur inhibant la croissance des cellules endothéliales humaines. Cet effet inhibiteur n'est pas dû à une activité cytotoxique mais à l'inhibition de la synthèse d'ADN. Le facteur en question, MECIF, est une glycoprotéine ayant un poids moléculaire apparent de 66 kd lorsqu'il est très purifié. L'expression de ce facteur dans les extraits monocytaires bruts est dépendante du nombre des monocytes ensemencés dans le puits de culture et sa production implique la synthèse protéique.

L'origine monocytaire du MECIF est confirmée par le fait que les cellules contaminantes possibles (plaquettes, lymphocytes), dans les conditions expérimentales utilisées, ne produisent aucune inhibition mais une faible proportion de lymphocytes (< 10 %) pourrait avoir une influence sur la production de l'inhibiteur par les monocytes.

On a montré que, de par ses propriétés biologiques, le MECIF isolé de l'extrait monocytaire brut est un facteur inhibiteur actif vis-à-vis de la croissance des cellules endothéliales. De plus, le MECIF provoque l'inhibition de la synthèse d'ADN dans les cellules endothéliales d'une manière dépendante de la dose. La puissance élevée du MECIF est démontrée par son aptitude à exercer ses effets à une concentration aussi faible que 10 pg/ml vis-à-vis de la croissance de 15000 cellules. De plus, on a trouvé que le MECIF est spécifique vis-à-vis des cellules, puisqu'il affecte peu la croissance des cellules, puisqu'il affecte peu la croissance des cellules des muscles lisses humains, des fibroblastes de peau humaine et des cellules endothéliales de porc. Toutefois, ces différentes cellules étaient à des stades différents de réplication et on ne peut par conséquent exclure la possibilité d'une réponse différente au MECIF selon l'âge de la cellule.

Les effets biologiques du MECIF sur la croissance des cellules endothéliales humaines pourraient être partiellement surmontés par la présence de quantités croissantes d'agents stimulant la croissance dans le milieu d'essai, tels que l'ECGF (facteur de croissance des cellules endothéliales).

La pureté du MECIF en tant que produit a été démontrée par l'apparition d'une bande protéique majeure unique en électrophorèse sur gel et également un pic unique après son passage à travers une colonne de chromatographie de filtration sur gel de type chromatographie liquide haute performance.

L'analyse par électrophorèse sur gel dans des conditions non réduites montre que la bande protéique majeure a un poids moléculaire apparent de 64 kd. Le MECIF présente des propriétés biologiques et chimiques différentes de celles de tous les produits régulant la croissance connus, d'origine monocytaire ou macrophagique, tels que les interférons (références 18,19,20), l'interleukine-1 (référence 11), le TNF (référence 21) ou le β TGF (référence 22).

Le MECIF diffère des autres facteurs inhibiteurs connus tels que les interférons, l'IL-1, le TNFα, le TGFβ par sa masse moléculaire apparente. Alors que son action a été similaire à celle du TGFβ sur la croissance des cellules endothéliales, de nombreuses caractéristiques différentes montrent que ce sont des facteurs différents. Le MECIF est synthétisé en l'absence de stimulation par une endotoxine tandis que le TGFβ requiert une telle stimulation. La réversibilité de la croissance des cellules endothéliales a été plus facile à obtenir après exposition des cellules endothéliales au MECIF qu'au TGFβ. Les anticorps spécifiques dirigés contre le TGFβ, le TNFα ("Cachetin") et les INF α et γ n'ont pas neutralisé les effets inhibiteurs du MECIF sur la croissance des cellules endothéliales.

La contamination possible du MECIF par des quantités infimes de TGFβ est apparue improbable étant donné que des préparations de MECIF à des concentrations plus élevées n'ont pas modifié la prolifération des fibroblasts de rein de rat normal qui sont des cellules de référence classiques pour le test biologique relatif au TGFβ.

D'autres facteurs inhibiteurs vis-à-vis de la croissance des cellules endothéliales de différents types cellulaires par leur origine, par exemple le cartilage (référence 23), l'humeur vitrée (référence 24), les plaquettes (référence 25), les cellules endothéliales (références 26 et 27) présentent également des propriétés différentes de celles du MECIF. En résumé, l'ensemble de ces études suggère que le MECIF est un facteur inhibiteur pour les cellules endothéliales qui diffère des autres facteurs inhibiteurs caractérisés auparavant (références 28 à 30).

Etant donné que l'on savait que les monocytes étaient capables de produire des agents favorisant la croissance des cellules endothéliales, le maintien de l'équilibre entre les facteurs favorisant et les facteurs inhibant la croissance devrait être important dans un grand nombre d'états pathophysiologiques comprenant l'inflammation et l'athérosclérose. De plus, cet équilibre pourrait jouer un rôle déterminant dans la modulation de l'angiogenèse qui peut accompagner dans les processus de réparation des tissus, les rétinopathies diabétiques et la néovascularisation tumorale.

### REFERENCES BIBLIOGRAPHIQUES

1. H. Setiadi, F. Lioté et J.L. Wautier. Monocytes and vascular lesions. Nouv. Rev. Fr. Hematol. (1986), 28, 339-343.
2. P.M. Henson, et R.B. Johnston Jr. Tissue injury in inflammation. Oxidants, proteinases, and cationic proteins. J. Clin. Invest. (1987), 79, 669-674.
3. L. Rifas, V. Shen, K. Mitchell, et W.A. Peck. Macrophage-derived growth factor for osteoblast-like cells and chondrocytes. Proc. Natl. Acad. Sci. U.S.A. (1984), 81, 4558-4562.
4. S.J. Leibovich, et R. Ross. A macrophage - dependent factor that stimulates the proliferation of fibroblasts in vitro. Am. J. Pathol. (1976), 84, 501-514.
5. K.C. Glenn, et R. Ross. Human monocyte-derived growth factor(s) for mesenchymal cells: activation of secretion by endotoxin and concanavalin A. Cell. (1981), 25, 603-615.
6. Y. Martinet, P.B. Bitterman, J.F. Mornex, G.R. Grotendorst, G.R. Martin, et R.G. Crystal. Activated human monocytes express the c-sis proto-oncogene and release a mediator showing PDGF-like activity. Nature. (1986), 319, 158-160.
7. P.J. Polverini, R.S. Cotran, M.A. Gimbrone, et E.R. Unanue. Activated macrophages induce vascular proliferation. Nature. (1977), 269, 804-806.
8. G.B. Greenburg, et T.K. Hunt. The proliferative response in vitro of vascular endothelial and smooth muscle cells exposed to wound fluids and macrophages J. Cell. Physiol. (1978), 96, 203-214.
9. R.T. Wall, L.A. Harker, L.J. Quadracci, et G.E. Striker. Factors influencing endothelial cell proliferation in vitro. J. Cell. Physiol. (1978), 96, 203-214.
10. B.M. Martin, M.A. Gimbrone, E.R. Unanue, et R.S. Cotran. Stimulation of nonlymphoid mesenchymal cell proliferation by a macrophage-derived growth factor. J. Immunol. (1981), 126, 1510-1515.
11. B.S. Ooi, E.P. Mc Carthy, A. Hsu, et Y.M. Ooi. Human mononuclear cell modulation of endothelial cell proliferation. J. Lab. Clin. Med. (1983), 102, 428-433.
12. A. Courillon Mallet, J.L. Wautier, et M.P. Wautier. Monocyte-derived endothelial cell inhibitory factor (MECIF). Sixth Inter-Washington Spring Symposium, ed. L.L. GALLO (1986), 160 a (abrégé).
13. M.B. Kahaleh, F. De Lustro, W. Bock, et E.D. LeRoy. Human monocyte modulation of endothelial cells and fibroblast growth: possible mechanism for fibrosis Clin. Immunol. Immunopathol. (1986), 39, 242-255.
14. J.L. Wautier, M.P. Wautier, D. Pintigny et coll. Factors involved in cell adhesion to vascular endothelium, Blood cells, (1983), 9, 221-234.
15. E. Engvall et P. Perlmann. Enzyme-linked immunosorbent assay (ELISA) III: quantification of specific antibodies by enzyme labelled anti-immunoglobulin in antigen coated tubes. J. Immunol. (1972), 109, 129-135.
16. H. Setiadi, J.L. Wautier, A. Courillon Mallet, P. Passa, et J.P. Caen. Increase adhesion to fibro-nectin and Mo-1 expression by diabetic monocytes. J. Immunol. (1987), 138, 3230-3234.
17. J.L. Wautier, D. Pintigny, J. Maclouf, M.P. Wautier, E. Corvazier et J.P. Caen. Release of prostacyclin after erythrocyte adhesion to cultured vascular endothelium. J. Lab. Clin. Med. (1986), 107, 210-215.
18. R. Friesel, A. Komoriya, et T. Maciag. Inhibition of endothelial cell proliferation by gamma-interferon. J. Cell. Biol. (1987), 104, 689-696.
10. W.E. Stewart, 2nd. Distinct molecular species of interferons. Virology (1974), 61, 80-86.
20. Y.K. Yip, B. Barrowclough, C. Urban, et J. Vilcek. The molecular weight of human gamma interferon is similar to that of other human interferons. Science (1982), 215, 411-413.
21. L.J. Old. Tumor necrosis factor. Polypeptide mediator network. Nature. (1987), 326, 330-331.
22. R.L. Heimark, D.R. Twardzik et S.M. Schwartz. Inhibition of endothelial regeneration by type-beta transforming growth factor from platelets. Science (1986), 233, 1078-1080.
23. H. Brem, et J. Folkman. Inhibition of tumor angiogenesis mediated by cartilage. J. Exp. Med. (1975) 141, 427-439.
24. L. Raymond, et B. Jacobson. Isolation and identification of stimulatory and inhibitory cell growth factors in bovine vitreous. Exp. Eye. Res. (1982), 34, 267-286.
25. M.T. Brown, et D.R. Clemmons. Platelets contain a peptide inhibitor of endothelial cell replication and growth. Proc. Natl. Acad. Sci. U.S.A. (1986), 83, 3321-3325.
26. C.H. Willems, G.C.B. Asteldi, P.G. De Groot, M.D. Janssen, M.D. Gonsalvez, W.P. Zeijlemaker, J.A. Van Mourik, et W.G. Van Aken. Media conditioned by cultured human vascular endothelial cell inhibit the growth of vascular smooth muscle cells. Exp. Cell. Res. (1982), 139, 191-197.
27. J.J. Castellot Jr., L.V. Faveau, M.J. Karnovsky, et R.D. Rosenberg. Inhibition of vascular smooth muscle cell growth by endothelial cell-derived heparin. Possible role of platelet endoglycosidase J. Biol. Chem. (1982), 257, 11256-11260.
28. R. Crum, S. Szabo, et J. Folkman. A new class of steroids inhibits angiogenesis in the presence of heparin or a heparin fragment. Science (1985), 230, 1375-1378.
29. R.L. Heimark et S.M. Schwartz. The role of membrane-membrane interactions in the regulation of endothelial cell growth. J. Cell. Biol. (1985), 100, 1934-1940.
30. B.L. Schumacher, D. Grant, et R. Eisenstein. Smooth muscle cells produce an inhibitor of endothelial cell growth. Arteriosclerosis. (1985), 5, 110-115.

## Revendications

1. Protéine, de préférence glycosylée, de poids moléculaire compris entre environ 0,6 et 200 kd qui présente un degré de pureté tel qu'il soit possible de l'analyser et qui ne modifie pas de manière sensible la croissance des cellules des muscles lisses, comportant la séquence :
- Ser - Pro - Glu - Leu - Thr - Phe -.

2. Protéine selon la revendication 1, caractérisée en ce que ladite séquence est N-terminale.

3. Protéine selon la revendication 1 ou 2, caractérisée en ce que ladite séquence est :
-Ser-Pro-Glu-Leu-Thr-Phe-Arg-X-Y-Thr-Ile-Ser-
où X et Y, qui peuvent être identiques ou différents, représentent chacun Trp ou Asp.

4. Protéine selon la revendication 3, caractérisée en ce que X et Y sont différents et en ce que de préférence X représente Trp et Y représente Asp.

5. Glycoprotéine de masse moléculaire apparente 64-70 kd, comportant 568 résidus d'acides aminés, qui se répartissent selon les proportions suivantes :
Asp : environ 80 : Thr : environ 23,06 : Ser : environ 29,40 : Glu : environ 56,5 : Pro : environ 27,70 : Gly : environ 38,98 : Ala : environ 42,88 : Val : environ 36,16 : Met : environ 4,83 : Ile : environ 18,79 : Leu : environ 50.92 : Tyr : environ 20,15 : Phe : environ 27,16 : GlcNH₂ : environ 3,10 : Lys : environ 60,87 : His : environ 13,81 : Arg : environ 27,60, et qui présente la séquence peptidique N-terminale :
Ser-Pro-Glu-Leu-Thr-Phe-Arg-X-Y-Thr-Ile-Ser
où X et Y, qui peuvent être identiques ou différents, représentent chacun Trp ou Asp.

6. Glycoprotéine selon la revendication 5, caractérisée en ce que sa masse moléculaire apparente est de 66 kd.

7. Procédé pour l'extraction de la glycoprotéine selon la revendication 5 ou 6, caractérisé en ce qu'il comprend essentiellement les étapes consistant à :
1°) cultiver des monocytes humains normaux,
2°) faire passer le surnageant de culture filtré à travers une colonne en polysaccharide réticulé, équilibrée à pH 8 environ,
3°) faire passer ensuite ce surnageant à travers une colonne de gel d'agarose phénylé et réticulé, équilibrée à pH 8 environ, en développant avec un gradient décroissant de sulfate d'ammonium,
4°) faire passer les fractions actives obtenues sous 3°) à travers une colonne du type de celle utilisée sous 2°), équilibrée à pH 8 environ,
5°) faire passer l'éluat obtenu sous 4°) à travers une colonne échangeuse d'ions de type diéthylaminoéthylée (DEAE), en développant avec un gradient croissant de chlorure de sodium,
6°) faire passer les fractions obtenues sous 5°) à travers une colonne de chromatographie d'exclusion basse pression, équilibrée au moyen d'un milieu de culture de cellules, et
7°) recueillir le produit purifié, éventuellement après avoir soumis les fractions actives obtenues sous 6°) à une chromatographie liquide haute pression.

8. Médicament, caractérisé en ce qu'il contient, en tant que principe actif, au moins une (glyco)protéine selon l'une des revendications 1 à 6.

9. Utilisation de la glycoprotéine selon la revendication 5 ou 6 pour la préparation d'anticorps.

10. Utilisation de la protéine selon l'une des revendications 1 à 4 pour la préparation d'anticorps.

## Claims

1. A preferably glycosylated protein having a molecular weight of between about 0.6 and 200 kD, which has a purity degree such that it be possible to analyze it and which does not appreciably modify the growth of smooth muscle cells, containing the sequence :
-Ser-Pro-Glu-Leu-Thr-Phe-.

2. A protein according to claim 1, characterized in that said sequence is N-terminal.

3. A protein according to claim 1 or 2, characterized in that said sequence is :
-Ser-Pro-Glu-Leu-Thr-Phe-Arg-X-Y-Thr-Ile-Ser,
wherein X and Y, which may be identical or different, each represent Trp or Asp.

4. A protein according to claim 3, characterized in that X and Y are different and in that, preferably, X represents Trp and Y represents Asp.

5. A glycoprotein with an apparent molecular weight of 64-70 kD, containing 568 amino acid residues distributed in the following proportions :
Asp: about 80 ; Thr: about 23.06 ; Ser: about 29.40 ; Glu: about 56.5 ; Pro: about 27.70 ; Gly: about 38.98 ; Ala: about 42.88 ; Val: about 36.16 ; Met: about 4.83 ; Ile: about 18.79 ; Leu: about 50.92 ; Tyr: about 20.15 ; Phe: about 27.16 ; GlcNH₂: about 3.10 ; Lys: about 60.87 ; His: about 13.81 ; Arg: about 27.60, and having the following N-terminal peptide sequence :
Ser-Pro-Glu-Leu-Thr-Phe-Arg-X-Y-Thr-Ile-Ser-,
wherein X and Y, which may be identical or different, each represent Trp or Asp.

6. A glycoprotein according to claim 5, characterized in that its apparent molecular weight is 66 kD.

7. A process for the extraction of the glycoprotein according to claim 5 or 6, characterized in that it essentially comprises the following steps :
1°) cultivating normal human monocytes,
2°) passing the filtered supernatant layer of culture through a column of cross-linked polysaccharide equilibrated at about pH 8,
3°) then passing this supernatant layer through a column of phenylated and cross-linked agarose gel equilibrated at about pH 8, employing the technique of decreasing gradient elution with ammonium sulphate,
4°) passing the active fractions obtained under 3°) through a column of the type used under 2°) equilibrated at about pH 8,
5°) passing the eluate obtained under 4°) through an ion exchange column of the diethylaminoethyl type (DEAE), employing the technique of increasing gradient elution with sodium chloride,
6°) passing the fractions obtained under 5°) through a low pressure exclusion chromatography column equilibrated by means of a cell culture medium, and
7°) collecting the purified product, possibly after having subjected the active fractions obtained under 6°) to a high pressure liquid chromatography.

8. A medicament, characterized in that it contains, as active principle, at least one (glyco)protein according to any one of claims 1 to 6.

9. Use of the glycoprotein according to claim 5 or 6 for the preparation of antibodies.

10. Use of the protein according to any one of claims 1 to 4 for the preparation of antibodies.

## Patentansprüche

1. Vorzugsweise glycosyliertes Protein mit einem Molekulargewicht zwischen etwa 0,6 und 200 kd, das einen solchen Reinheitsgrad aufweist, daß seine Analyse möglich ist, und daß das Wachstum von Zellen glatter Muskeln nicht wahrnehmbar verändert, umfassend die Sequenz:
- Ser - Pro - Glu - Leu - Thr - Phe - .

2. Protein gemäß Anspruch 1, dadurch gekennzeichnet, daß die Sequenz N-terminal ist.

3. Protein gemäß Anspruch 1 oder 2, dadurch gekennzeichnet, daß die Sequenz folgende ist:
-Ser-Pro-Glu-Leu-Thr-Phe-Arg-X-Y-Thr-Ile-Ser-
worin X und Y, die gleich oder verschieden sein können, jeweils Trp oder Asp bedeuten.

4. Protein gemäß Anspruch 3, dadurch gekennzeichnet, daß X und Y verschieden sind, und daß vorzugsweise X Trp, und Y Asp bedeuten.

5. Glycoprotein mit einer scheinbaren Molekularmasse von 64 bis 70 kd, umfassend 568 Aminosäurereste, die sich gemäß folgenden Anteilen aufteilen:
Asp: etwa 80; Thr : etwa 23,06; Ser: etwa 29,40; Glu : etwa 56,5; Pro : etwa 27,70; Gly : etwa 38,98; Ala : etwa 42,88; Val : etwa 36,16; Met : etwa 4,83; Ile : etwa 18,79; Leu : etwa 50,92; Tyr : etwa 20,15; Phe : etwa 27,16; GlcNH₂ : etwa 3,10; Lys : etwa 60,87; His : etwa 13,81; Arg : etwa 27,60, und das die N-terminale Peptidsequenz
Ser-Pro-Glu-Leu-Thr-Phe-Arg-X-Y-Thr-Ile-Ser-
aufweist, worin X und Y, die gleich oder verschieden sein können, jeweils Trp oder Asp bedeuten.

6. Glycoprotein gemäß Anspruch 5, dadurch gekennzeichnet, daß seine scheinbare Molekularmasse 66 kd beträgt.

7. Verfahren zur Extraktion des Glycoproteins gemäß Anspruch 5 oder 6, dadurch gekennzeichnet, daß es im wesentlichen folgende Stufen umfaßt:
1.) Kultivierung von normalen menschlichen Monocyten,
2.) Durchleiten des Überstands der filtrierten Kultur durch eine auf einen pH-Wert von etwa 8 eingestellte Säule aus vernetztem Polysaccharid,
3.) nachfolgendes Durchleiten dieses Überstands durch eine auf einen pH-Wert von etwa 8 eingestellte Säule aus einem phenylierten und vernetztem Agarosegel, wobei mit einem fallenden Gradienten Ammoniumsulfat entwickelt wird,
4.) Durchleiten der in Stufe 3.) erhaltenen aktiven Fraktionen durch eine auf einen pH-Wert von etwa 8 eingestellte Säule von derart, wie sie in Stufe 2.) benutzt wurde,
5.) Durchleiten des in Stufe 4.) erhaltenen Eluats durch eine Ionenaustauschersäule vom Diethylaminoethyl- (DEAE)-Typ, wobei mit einem steigenden Gradienten Natriumchlorid entwickelt wird,
6.) Durchleiten der in Stufe 5.) erhaltenen Fraktionen durch eine Niederdruck-Ausschlußchromatographiesäule, die mittels eines Zellkulturmediums ins Gleichgewicht gebracht wurde, und
7.) Auffangen des gereinigten Produkts, ggfls. nachdem die in Stufe 6.) erhaltenen aktiven Fraktionen einer Hochdruck-Flüssigkeitschromatographie unterworfen wurden.

8. Arzneimittel, gekennzeichnet durch einen Gehalt an zumindest einem (Glyco)protein gemäß einem der Ansprüche 1 bis 6 als Wirkstoff.

9. Verwendung des Glycoproteins gemäß Anspruch 5 oder 6 zur Herstellung von Antikörpern.

10. Verwendung des Proteins gemäß einem der Ansprüche 1 bis 4 zur Herstellung von Antikörpern.
